# FASCICULE DE BREVET EUROPEEN

(11) **EP 3 883 519 B1**
(45) Date de publication et mention de la délivrance du brevet: **13.03.2024**
(21) Numéro de dépôt: 19831787.7
(22) Date de dépôt: 19.11.2019
(51) Int. Cl.: A61J 7/02, B65D 83/04, A61J 7/00

(54) **DISPOSITIF SECURISE DE COMPTAGE ET DE DISTRIBUTION D'OBJETS**
GESICHERTE VORRICHTUNG ZUM ZÄHLEN UND AUSGEBEN VON GEGENSTÄNDEN
SECURE DEVICE FOR COUNTING AND DISPENSING OBJECTS

(30) Priorité: 20.11.2018 FR 1871627
(43) Date de publication de la demande: 29.09.2021
(73) Titulaire: Stiplastics, 38160 Saint-Marcellin (FR)
(72) Inventeur: ZOCZEK, Guillaume, 38210 TULLINS (FR); SOUAL, Ludovic, 26100 ROMANS SUR ISERE (FR); BROUARD, Hugues, 26300 ROCHEFORT SAMSON (FR)
(74) Mandataire: Regimbeau
(86) Numéro de dépôt international: PCT/FR2019/052745
(87) Numéro de publication internationale: WO 2020/104748

(56) Documents cités:
- EP-A1- 0 051 994
- EP-B1- 0 051 994
- WO-A1-2005/044702
- US-A1- 2014 339 250

## Description

### DOMAINE DE L'INVENTION

La présente invention concerne un dispositif sécurisé de comptage et de distribution d'objets.

### ETAT DE LA TECHNIQUE

La demanderesse a conçu un dispositif de comptage et de distribution d'objets, notamment des granules homéopathiques, des gélules, des comprimés, des capsules ou des micro-granules, décrit dans le document WO 2015/121353.

Ce dispositif, qui est destiné à être couplé à un récipient contenant les objets à distribuer, comprend deux éléments mobiles en coulissement l'un par rapport à l'autre :
- un premier élément comprenant un conduit de distribution des objets à compter et distribuer,
- le second élément coopérant avec le premier pour former deux obturateurs délimitant dans le conduit de distribution une chambre adaptée pour contenir un nombre déterminé desdits objets.

Les obturateurs sont chacun configurés pour adopter, selon la position relative du premier et du second élément :
- une configuration dite d'ouverture de la chambre, dans laquelle chaque obturateur définit un orifice de dimension adaptée au passage d'un objet à compter et distribuer, et
- une configuration dite d'obturation de la chambre, dans laquelle ledit orifice présente une dimension insuffisante pour le passage d'un objet.

Chaque obturateur présente deux portions biseautées en vis-à-vis non jointives dans la configuration d'obturation, de sorte que l'orifice n'est jamais entièrement fermé. Ceci permet d'une part d'éviter de coincer un objet dans l'obturateur, ce qui serait susceptible d'endommager l'objet, et d'autre part d'éviter de cisailler un objet en contact avec l'obturateur lors de la fermeture de celui-ci.

Pour libérer un ou plusieurs objets, un utilisateur actionne le dispositif en exerçant un effort de coulissement relatif du premier et du second élément, ce qui produit la séquence de fonctionnement suivante :
(i) un premier obturateur est en configuration d'ouverture tandis que le second obturateur est en configuration d'obturation de la chambre,
(ii) le premier et le second obturateurs sont tous deux en configuration d'obturation de la chambre,
(iii) le premier obturateur est en configuration d'obturation tandis que le second obturateur est en configuration d'ouverture de la chambre,
(iv) le premier et le second obturateurs sont tous deux en configuration d'obturation de la chambre.

Dans certaines applications, un certain degré de sécurisation de ce dispositif serait souhaitable, afin de ne permettre la libération des objets que dans des conditions déterminées.

Par exemple, la médecine personnalisée implique la prise, par un patient, d'une dose déterminée et possiblement évolutive de médicament à un moment déterminé, une dose se présentant sous la forme d'un ou plusieurs des objets susmentionnés.

Pour éviter que le patient n'ingère une dose inadaptée du médicament, ou qu'il n'ingère le médicament à un moment non prévu, il faudrait que le dispositif soit pilotable pour empêcher la libération des objets en dehors d'une plage temporelle déterminée, et qu'il ne délivre que la dose prescrite ou autorisée pendant cette plage.

Par ailleurs, il peut être nécessaire de contrôler l'observance du traitement, c'est-à-dire de vérifier que le patient a bien pris la dose prévue.

Une autre contrainte liée à la sécurité est d'éviter qu'un corps étranger ne puisse être introduit dans le récipient au travers du dispositif de distribution, afin d'éviter la contamination des objets ou la présence de substances étrangères au traitement parmi les objets.

EP 0 051 994 divulgue un dispositif de comptage et de distribution d'objets comprenant deux éléments mobiles en coulissement l'un par rapport à l'autre et deux obturateurs.

### EXPOSE DE L'INVENTION

Un but de l'invention est donc de concevoir un dispositif de comptage et de distribution d'objets qui soit sécurisé.

A cet effet, l'invention propose un dispositif de comptage et de distribution d'objets, comprenant deux éléments mobiles en coulissement l'un par rapport à l'autre,
- un premier élément comprenant un conduit de distribution des objets à compter et distribuer, ledit conduit s'étendant dans la direction de coulissement,
- le second élément coopérant avec le premier pour former deux obturateurs délimitant dans le conduit de distribution une chambre adaptée pour contenir un nombre déterminé desdits objets,

lesdits obturateurs étant aptes à adopter, selon la position relative desdits premier et second éléments :
   - une configuration dite d'ouverture de la chambre, dans laquelle chaque obturateur définit un orifice de dimension adaptée au passage d'un objet à compter et distribuer, et
   - une configuration dite d'obturation de la chambre, dans laquelle ledit orifice présente une dimension insuffisante pour le passage d'un objet, chaque obturateur présentant deux portions biseautées en vis-à-vis non jointives dans ladite configuration d'obturation,
les premier et second éléments étant agencés pour procurer, par coulissement relatif, une séquence de fonctionnement des obturateurs dans laquelle :
   (i) un premier obturateur est en configuration d'ouverture tandis que le second obturateur est en configuration d'obturation de la chambre,
   (ii) le premier et le second obturateurs sont tous deux en configuration d'obturation de la chambre,
   (iii) le premier obturateur est en configuration d'obturation tandis que le second obturateur est en configuration d'ouverture de la chambre,
   (iv) le premier et le second obturateurs sont tous deux en configuration d'obturation de la chambre,
ledit dispositif étant caractérisé en ce qu'il est adapté pour être inséré dans un boîtier comprenant une unité de contrôle et au moins un actionneur piloté par ladite unité de contrôle, et en ce qu'il comprend un verrou déplaçable par l'actionneur entre un état de verrouillage dans lequel ledit verrou obture partiellement le conduit de distribution, et un état de déverrouillage dans laquelle le verrou est escamoté hors du conduit de distribution, ledit verrou étant configuré pour que la position d'un objet présent dans la chambre ne soit pas affectée par un changement d'état du verrou.

De manière particulièrement avantageuse, le verrou est agencé dans un même plan que les portions biseautées du second obturateur.

Selon un mode de réalisation, le verrou comprend deux palettes pivotantes adaptées pour être couplées à un actionneur respectif.

Selon une forme d'exécution avantageuse, le premier élément, le second élément et le verrou sont chacun réalisés d'un seul tenant, le dispositif étant uniquement constitué desdits premier et second éléments et du verrou.

Un autre objet de l'invention concerne un récipient inviolable comprenant un tel dispositif de distribution et un pot contenant les objets, ledit pot étant couplé de manière inamovible au dispositif de distribution.

Lesdits objets peuvent être des granules homéopathiques, des gélules, des comprimés, des capsules conditionnés en vrac dans le pot.

Un autre objet de l'invention concerne un distributeur sécurisé d'une dose d'un traitement thérapeutique se présentant sous la forme d'un ou plusieurs objets, ledit distributeur comprenant un boîtier et un récipient tel que décrit ci-dessus coopérant avec ledit boîtier, dans lequel le boîtier comprend :
- une unité de contrôle comprenant un processeur et une mémoire dans laquelle sont enregistrées des données relatives au traitement,
- un actionneur piloté par ladite unité de contrôle pour déplacer le verrou dans sa position de verrouillage ou dans sa position escamotée.

Selon un mode de réalisation, l'actionneur est un actionneur linéaire.

De manière alternative, l'actionneur est un actionneur rotatif.

Selon un mode de réalisation, le boîtier comprend au moins un capteur couplé à l'unité de contrôle et adapté pour détecter le passage d'un objet dans la chambre et/ou dans une portion du conduit située en aval du second obturateur.

Par ailleurs, le boîtier peut comprendre au moins un capteur couplé à l'unité de contrôle et adapté pour détecter la configuration d'ouverture ou d'obturation de chaque obturateur.

Le boîtier peut comprendre en outre un dispositif d'identification du patient couplé à l'unité de contrôle, l'unité de contrôle étant configurée pour activer l'actionneur seulement si le patient est identifié par le dispositif d'identification.

Ledit dispositif d'identification peut comprendre un dispositif biométrique ou une interface de saisie d'un code d'identification.

Selon un mode de réalisation, le distributeur comprend un accéléromètre ou un gyroscope couplé à l'unité de contrôle, dans lequel l'unité de contrôle est configurée pour déterminer l'orientation du distributeur à partir des données de mesure dudit accéléromètre ou gyroscope et pour activer l'actionneur uniquement si le conduit de distribution est orienté sensiblement verticalement et que la sortie du conduit de distribution est dirigée vers le bas.

Le distributeur peut également comprendre un capteur de température et une mémoire configurée pour enregistrer les données de mesure dudit capteur.

De manière avantageuse, le boîtier comprend une source d'énergie alimentant l'unité de contrôle.

Selon un mode de réalisation avantageux, le boîtier comprend une base sensiblement plane adaptée pour être posée sur un support lors de la distribution des objets.

### BREVE DESCRIPTION DES FIGURES

D'autres caractéristiques et avantages de l'invention ressortiront de la description détaillée qui va suivre, en référence aux dessins annexés sur lesquels :
- la figure 1 illustre différents exemples d'objets susceptibles d'être distribués par le dispositif selon l'invention,
- la figure 2A illustre de manière schématique une première configuration des obturateurs d'un dispositif selon l'invention,
- la figure 2B illustre de manière schématique une deuxième configuration des obturateurs d'un dispositif selon l'invention,
- la figure 2C illustre de manière schématique une troisième configuration des obturateurs d'un dispositif selon l'invention,
- la figure 2D illustre de manière schématique une quatrième configuration des obturateurs d'un dispositif selon l'invention,
- la figure 3A représente une vue de côté de l'élément fixe du dispositif de distribution selon un mode de réalisation,
- la figure 3B représente une vue en perspective de l'élément fixe du dispositif de distribution selon un mode de réalisation,
- la figure 3C représente une vue en coupe du dispositif de distribution incluant l'élément fixe des figures 3A et 3B et l'élément mobile, dans une orientation permettant la distribution d'un objet,
- la figure 4A est une vue en perspective d'un premier mode de réalisation du verrou, à l'état escamoté,
- la figure 4B est une vue de dessous du verrou à l'état escamoté de la figure 4A,
- la figure 4C est une vue en perspective dudit verrou à l'état escamoté,
- la figure 4D est une vue de dessous du verrou à l'état escamoté de la figure 4C,
- la figure 5A est une vue en perspective d'un second mode de réalisation du verrou, à l'état escamoté,
- la figure 5B est une vue de dessous du verrou à l'état escamoté de la figure 5A,
- la figure 5C est une vue en perspective dudit verrou à l'état escamoté,
- la figure 5D est une vue de dessous du verrou à l'état escamoté de la figure 5C,

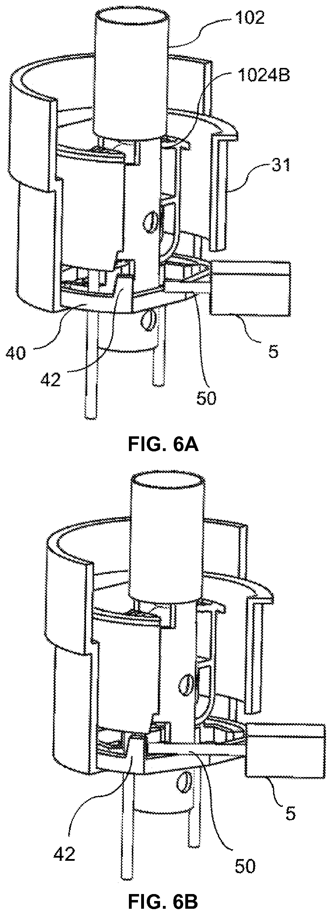

- la figure 6A illustre la coopération du verrou avec un actionneur, à l'état escamoté,
- la figure 6B illustre la coopération du verrou avec l'actionneur, à l'état verrouillé,
- la figure 7 représente des guides de lumière agencés dans le dispositif de distribution pour coopérer avec des capteurs optiques,
- la figure 8 est une vue du dispositif de comptage et de distribution et du boîtier avant assemblage,
- la figure 9 est une vue du dispositif de comptage et de distribution et du boîtier après assemblage.

Il va de soi que ces figures représentent des modes de réalisation donnés à titre d'illustration, mais que l'invention n'est en aucun cas limitée à la géométrie et l'agencement des composants ainsi représentés.

### DESCRIPTION DETAILLEE DE MODES DE REALISATION

### Objets à compter et à distribuer

L'invention s'applique de manière générale au comptage et à la distribution de tout objet présentant une forme sphérique ou sphéroïdale ou encore une forme allongée avec des extrémités convexes. Selon un mode de réalisation non limitatif, les objets peuvent présenter une symétrie de révolution par rapport à un axe qui s'étend entre les deux extrémités convexes.

Dans le présent texte, on désigne par « allongé » un objet dont la plus grande dimension (ou longueur) s'étend entre les deux extrémités convexes.

Les objets allongés sont destinés à être orientés dans un conduit du dispositif de comptage et de distribution les uns à la suite des autres dans le sens de leur plus grande dimension.

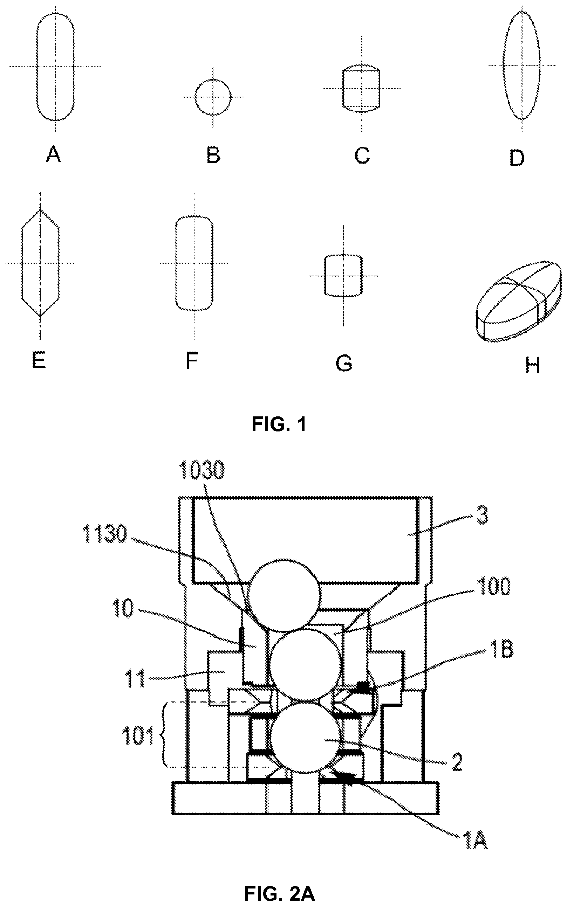

La figure 1 illustre quelques exemples de tels objets, désignés de A à H.

Une gélule est représentée sur la figure 1A. Ladite gélule peut être définie comme présentant une portion cylindrique de section circulaire et deux extrémités hémisphériques symétriques, dont le rayon de courbure est égal au rayon de la portion cylindrique. La distance entre les deux extrémités hémisphériques est supérieure au diamètre de la portion circulaire.

La figure 1B illustre un objet sphérique, tel qu'un granule.

La figure 1C illustre un micro-granule, qui est un micro-comprimé, de diamètre variant typiquement de 0,8 à 4 mm, présentant une section cylindrique constante sur une longueur typiquement égale au diamètre et présentant des extrémités dont la section va en se rétrécissant, typiquement en forme de calotte sphérique.

Une capsule est représentée sur la figure 1D. Ladite capsule peut être définie comme un ellipsoïde de révolution.

La figure 1E illustre un objet présentant une portion cylindrique de section circulaire et deux extrémités pointues symétriques. La distance entre les extrémités pointues est supérieure au diamètre de la portion cylindrique.

La figure 1F illustre un objet présentant une portion cylindrique de section circulaire et deux extrémités arrondies symétriques. Le rayon de courbure peut être plus ou moins grand mais le cas où les extrémités sont planes est exclu.

La figure 1G présente un cas limite où la variation des sections des extrémités diminue très rapidement, sans toutefois être un cylindre à section constante, et pour lequel des dispositifs selon l'invention sont performants.

La figure 1H illustre le cas d'un comprimé qui, contrairement aux objets des figures 1A à 1G, ne présente pas de symétrie de révolution. Cet objet peut être défini comme présentant une portion droite cylindrique dont la base est une ellipse et deux faces en forme d'ellipsoïde. Les extrémités de l'objet correspondent aux extrémités du grand axe de l'ellipse.

D'une manière générale, toutes les formes obtenues dans le domaine de la galénique solide (à l'exception des poudres, dont les particules ne sont pas considérées comme des objets à compter), peuvent s'appliquer aux objets à distribuer par le dispositif de l'invention.

Dans les applications pharmaceutiques, les objets peuvent être des granules, des micro-granules, des capsules, des comprimés, des ovules ou encore des gélules.

Cependant, tout objet présentant l'une des variantes de formes décrites ci-dessus, quelles que soient ses dimensions et proportions, peut être distribué en nombre déterminé au moyen d'un dispositif selon l'invention. L'invention trouve donc à s'appliquer de manière générale dans tout domaine de l'industrie, incluant l'agroalimentaire, dans lequel il est nécessaire de distribuer un nombre déterminé d'objets.

### Dispositif de comptage et de distribution

Le dispositif de comptage et de distribution des objets est similaire à celui déjà décrit dans le document WO 2015/121353, auquel on pourra se reporter pour la description de différents modes de réalisation.

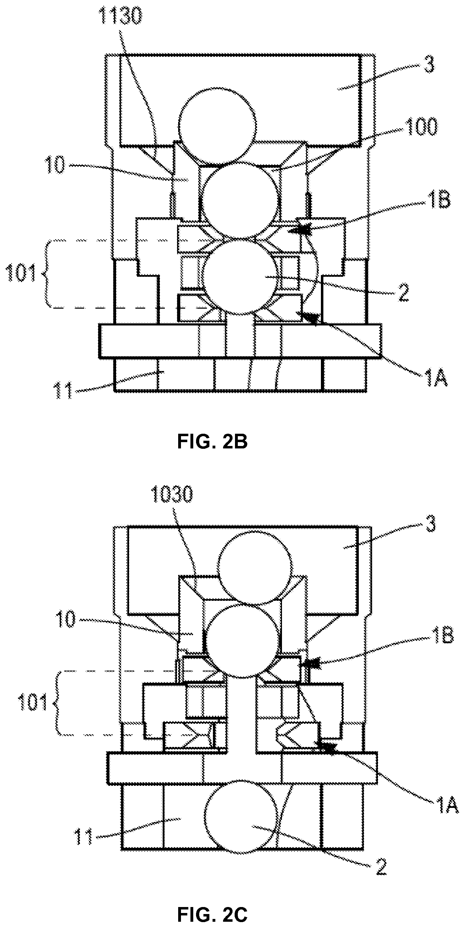

En référence aux figures 2A à 2D, on rappellera le principe du dispositif de comptage et de distribution (appelé dans la suite du texte « dispositif de distribution » dans un souci de concision).

Sur ces figures, le réservoir 3 d'objets 2 (représentés ici sous la forme de billes) est situé dans la partie supérieure du dispositif de distribution et communique avec un conduit 100 de distribution des objets qui s'étend selon un axe longitudinal X. Le sens d'écoulement des objets du réservoir 3 vers la sortie du dispositif de distribution est représenté par la flèche. Pour la distribution des objets, on oriente l'axe X dans une direction verticale ou oblique, avec le réservoir au-dessus du dispositif de distribution, pour permettre aux objets de s'écouler par gravité dans le conduit 100.

Le dispositif de distribution comprend deux éléments mobiles en coulissement l'un par rapport à l'autre :
- un premier élément 10 comprenant le conduit 100,
- le second élément 11 coopérant avec le premier pour former deux obturateurs 1A, 1B délimitant dans le conduit de distribution 100 une chambre 101 adaptée pour contenir un nombre déterminé desdits objets.

Dans la présente invention, on envisage uniquement un coulissement des deux éléments dans la direction de l'axe X du conduit de distribution.

Le conduit 100 présente, perpendiculairement à l'axe X, une section adaptée pour le passage d'un seul objet, de sorte que les objets sont superposés dans le conduit 100.

Si les objets à distribuer sont des objets allongés, la section du conduit 100 est adaptée pour le passage des objets dans le sens de leur longueur, c'est-à-dire que les objets sont superposés dans le conduit 100 avec leurs extrémités convexes en vis-à-vis. La section du conduit 100 ne permet pas le passage d'un objet dans une autre orientation que celle de sa longueur, ni le passage simultané de deux objets ou davantage au travers d'une section donnée du conduit.

De manière avantageuse, la jonction entre le réservoir d'objets et le dispositif de distribution présente la forme d'un entonnoir qui permet d'éviter un arc-boutement des objets à l'entrée du conduit 100. En effet, les objets sphériques ou allongés avec des extrémités convexes, compte tenu de leurs symétries, peuvent tendre à s'organiser de telle sorte que, étant prélevés par le centre du fond d'un réservoir, ils forment eux-mêmes une structure stable, pseudo-tubulaire et donc creuse en son centre, qui ne peut s'affaisser, pour que le prélèvement puisse continuer, que sous l'action d'un brassage mécanique.

La section intérieure du réservoir 3 est généralement supérieure à celle du conduit 100 et la paroi intérieure du réservoir comprend avantageusement une portion 1130 inclinée vers l'aval dans le sens d'un rétrécissement de la section. L'extrémité amont du conduit 100 présente quant à elle une paroi 1030 qui est inclinée dans le même sens que la portion 1130.

Les obturateurs 1A et 1B sont situés respectivement dans des parties aval et amont du conduit 100 par rapport au sens d'écoulement des objets pour sortir du dispositif de distribution.

Les obturateurs 1A et 1B délimitent entre eux une portion du conduit 100 qui est une chambre 101 destinée à contenir un nombre déterminé d'objets. Dans l'exemple illustré sur les figures 2A à 2D, la chambre est destinée à recevoir un unique objet, c'est-à-dire que la distance entre les obturateurs 1A et 1B selon l'axe X est égal au diamètre nominal de l'objet, mais inférieure à 1,5 fois le diamètre nominal dudit objet. Cependant, le dispositif de distribution selon l'invention peut être conçu pour que la chambre 101 comprenne deux objets ou davantage ; il suffit pour cela d'adapter la distance entre les obturateurs 1A et 1B. La chambre 101 remplit ainsi la fonction de comptage des objets à distribuer tout en offrant la possibilité d'absorber les tolérances dimensionnelles inhérentes aux procédés de fabrication desdits objets.

La figure 2A illustre un état du dispositif de distribution dans lequel les deux obturateurs 1A, 1B sont tous les deux en configuration d'obturation de la chambre 101, qui est alors vide.

On notera que les portions en vis-à-vis des obturateurs 1A, 1B ne sont pas jointives mais distantes d'un intervalle inférieur au diamètre d'un objet, de sorte à bloquer le passage d'un objet 2 présent dans le conduit directement en amont de l'obturateur 1B. Comme on le verra plus bas, le fait que les obturateurs ne soient pas jointifs présente l'avantage de permettre de retenir les objets sans exercer de cisaillement sur un objet contenu dans la chambre 101. Par ailleurs, les portions en vis-à-vis des obturateurs présentent avantageusement un profil biseauté.

Le fait que l'obturateur 1B soit biseauté permet que lors du mouvement de fermeture dudit obturateur l'on évite d'exercer une contrainte de cisaillement sur l'objet contenu dans la chambre 101 ou sur l'objet situé directement en amont dudit obturateur 1B.

Enfin, la figure 2D illustre un état du dispositif de distribution dans lequel l'obturateur amont 1B reste en configuration de fermeture de la chambre 101 tandis que l'obturateur aval 1A est en configuration d'ouverture de ladite chambre. Le passage de l'état de la figure 2C à celui de la figure 2D est obtenu par un coulissement relatif des deux éléments du dispositif de distribution. Ce coulissement est opéré dans la direction de l'axe X, de l'aval vers l'amont. On notera que ce coulissement a pour effet de faire pénétrer l'élément 10 dans le réservoir 3, produisant ainsi un brassage des objets qui évite très efficacement un éventuel arc-boutement des objets à l'entrée du réservoir.

L'ouverture de l'obturateur aval 1A permet à l'objet 2 (ou aux objets si deux objets ou davantage étaient contenus dans la chambre 101) de s'échapper de la chambre et d'être ainsi extrait du dispositif de distribution.

Comme mentionné plus haut, la conformation des obturateurs, à savoir le fait qu'ils ne soient pas jointifs en configuration d'obturation et qu'ils présentent un profil biseauté, permet, lors de la fermeture de l'obturateur d'éviter d'exercer une contrainte de cisaillement sur un objet inséré dans la chambre 101 ou sur un objet en amont de l'obturateur 1B qui se serait partiellement engagé dans la chambre 101 et ce, même si la dimension des objets varie dans une gamme déterminée. Par ailleurs, cette conformation de l'obturateur permet de repousser vers l'amont, sans exercer de contrainte de cisaillement susceptible de l'endommager, un objet surnuméraire partiellement engagé dans la chambre 101.

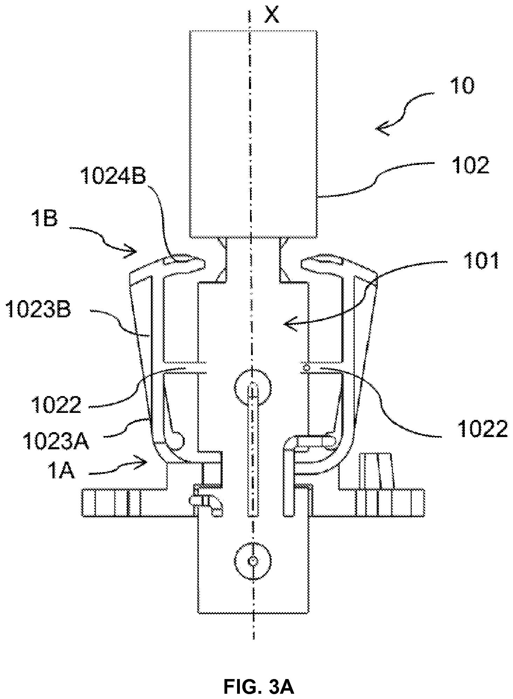

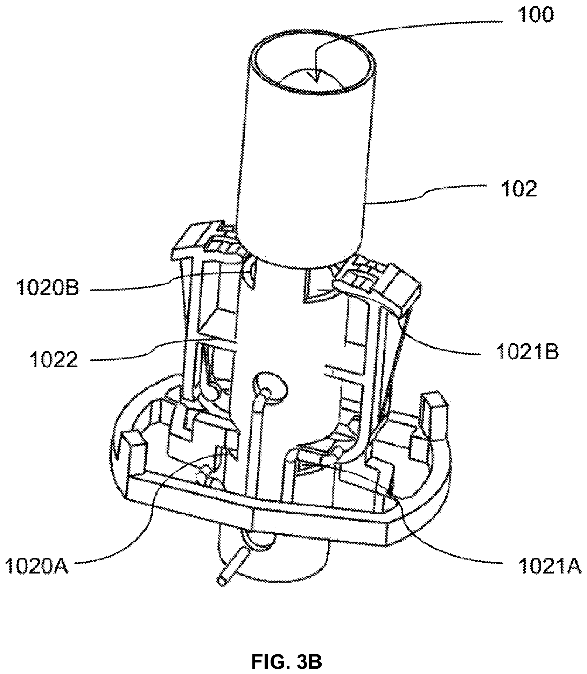

Les figures 3A et 3B sont respectivement une vue de côté et une vue en perspective d'un mode de réalisation de l'élément 10 comprenant le conduit de distribution. L'élément est représenté orienté verticalement, dans une position qui permet de faire sortir les objets par le bas. Les termes « inférieur » et « supérieur » ou « haut » et « bas » employés ci-après s'entendent par rapport à cette position.

L'élément 10 comprend un fût central 102 à l'intérieur duquel est agencé le conduit de distribution 100. Le fût central présente deux paires d'ouvertures 1020B-1021B et 1020A-1021A diamétralement opposées, délimitant la partie amont et la partie aval de la chambre de comptage 101. Le fût central 102 comprend également deux pieds 1022 diamétralement opposés s'étendant radialement vers l'extérieur. Chaque pied supporte deux bras 1023B, 1023A qui s'étendent dans une direction sensiblement parallèle au fût 102, de part et d'autre du pied 1022. Chaque bras se termine par une extension s'étendant radialement vers l'intérieur, en regard d'une ouverture respective 1020B, 1021B, 1020A, 1021A dans le fût 102. Cette extension est désignée par le repère 1024B pour le bras 1023B, celle du bras 1023A n'étant pas visible car positionnée à l'intérieur de l'ouverture 1020A. Chaque ensemble constitué d'un pied 1022 et de ses deux bras est symétrique de l'autre par rapport à un plan comprenant l'axe X.

Compte tenu de la forme et du matériau polymère employé pour former le pied et les bras, les bras présentent une certaine élasticité en pivotement autour de leur jonction avec le pied. Les bras supérieurs 1023B sont donc mobiles entre une position où les extensions supérieures 1024B sont rapprochées et pénètrent partiellement dans le conduit de distribution 100 au travers des ouvertures supérieures 1020B, 1021B et une position où les extensions supérieures 1024B sont éloignées l'une de l'autre et situées en-dehors du conduit de distribution 100, libérant ainsi le passage des objets. En d'autres termes, les extensions supérieures 1024B forment ainsi un obturateur amont 1B de la chambre de comptage 101.

De même, les bras inférieurs 1023A sont mobiles entre une position où les extensions inférieures sont rapprochées et pénètrent partiellement dans le conduit de distribution 100 au travers des ouvertures inférieures 1020A, 1021A et une position où les extensions inférieures sont éloignées l'une de l'autre et situées en-dehors du conduit de distribution 100, libérant ainsi le passage des objets. En d'autres termes, les extensions inférieures forment ainsi un obturateur aval 1A de la chambre de comptage 101.

Comme expliqué plus haut, les extensions ne sont pas jointives même dans leur position rapprochée, et présentent une extrémité biseautée qui permet de ne pas appliquer de contrainte de cisaillement à un objet lors de leur rapprochement.

Les figures 3A et 3B représentent le premier élément en configuration de repos, c'est-dire une configuration dans laquelle aucune sollicitation mécanique n'est appliquée aux bras. Dans cette configuration, les bras supérieurs 1024B sont dans une position éloignée l'une de l'autre, de sorte que les extensions 1024B définissent un orifice suffisamment large pour le passage d'un objet. L'obturateur amont 1B est donc en configuration d'ouverture de la chambre 101. En revanche, les bras inférieurs 1024A sont dans une position rapprochée l'une de l'autre, de sorte que les extensions 1024A, bien que non jointives, définissent un orifice suffisamment étroit pour empêcher le passage d'un objet. L'obturateur aval 1A est donc en configuration d'obturation de la chambre 101.

Le second élément du dispositif est conformé pour, selon sa position par rapport au premier élément, rapprocher ou éloigner les bras supérieurs et inférieurs afin de procurer la séquence de fonctionnement décrite plus haut.

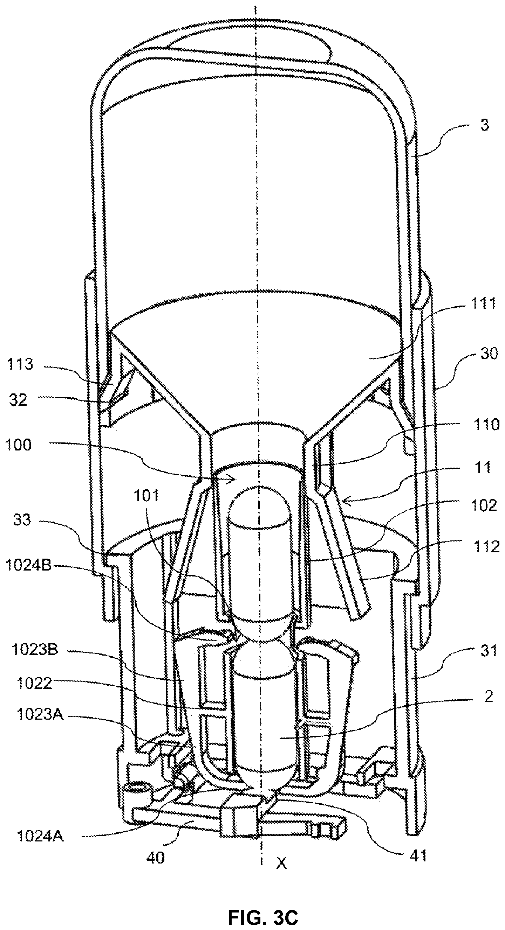

Ce second élément 11 est illustré sur la figure 3C assemblé avec l'élément fixe 10 des figures 3A et 3B et avec un récipient 3 contenant les objets à distribuer.

L'élément fixe est rigidement solidaire d'un corps 31 qui enferme les composants de l'élément 10 de sorte à les rendre inaccessibles pour un utilisateur.

Le récipient 3 est monté en coulissement dans la direction X par rapport au corps 31 par l'intermédiaire d'un raccord tubulaire 30. Le corps 31 présente une collerette supérieure 33 qui constitue une butée axiale vers le bas pour le récipient 3.

L'élément 11 comprend une portion cylindrique 110 agencée en coulissement sur le fût 102.

Du côté amont, c'est-à-dire orienté vers l'intérieur du récipient 3, la portion cylindrique 110 se prolonge avantageusement par un entonnoir 111 qui débouche dans le conduit de distribution 100. Un tel entonnoir, bien qu'optionnel, permet d'éviter l'arc-boutement des objets à l'entrée du conduit de distribution 100.

Du côté aval, c'est-à-dire orienté vers l'obturateur aval 1A, la portion cylindrique 110 se prolonge par une jupe 112 qui s'évase en direction de l'aval.

De manière avantageuse, la portion cylindrique 110, l'entonnoir 111 et la jupe 112 forment une unique pièce, qui peut être réalisée par moulage d'une matière plastique.

L'élément 11 est rigidement solidaire du récipient 3. Par exemple, dans le mode de réalisation illustré, la partie formant entonnoir 111 comprend un ou plusieurs ergots 113 encliquetés dans des ouvertures 32 formées sur la circonférence du récipient 3. Ainsi, l'élément 11 est entraîné en coulissement avec le récipient 3 lorsqu'un effort de poussée est exercé sur celui-ci par un utilisateur.

La surface intérieure de la jupe est configurée pour coopérer avec les bras afin d'actionner les obturateurs 1A, 1B. Ainsi, selon la position de la jupe par rapport aux bras, la jupe est susceptible d'exercer une sollicitation mécanique sur les bras 1023B et 1023A de sorte à déformer élastiquement lesdits bras en vue de les rapprocher ou de les éloigner.

Plus précisément, le dispositif est configuré pour procurer la séquence de fonctionnement suivante.

Dans une configuration de repos, dans laquelle aucune action n'est effectuée par un utilisateur pour tenter de libérer un ou des objets, les premier et second éléments 10, 11 sont dans la position relative illustrée sur la figure 1C. Dans cette position, la jupe 112 est éloignée des bras supérieurs 1023A vers le haut et n'exerce donc aucune sollicitation mécanique sur lesdits bras. Comme indiqué plus haut, l'obturateur 1B est donc en configuration d'ouverture de la chambre 101, tandis que l'obturateur 1A est en configuration d'obturation de la chambre. Un objet (sous la forme d'une gélule dans l'application illustrée) est donc contenu dans la chambre 101 entre les deux obturateurs.

Pour libérer ledit objet, l'utilisateur doit exercer une pression sur le récipient 3 selon l'axe X en direction de l'aval. Ce faisant, le second élément 11 est entraîné en coulissement vers le bas le long du fût 102. La surface intérieure de la jupe 112 entre donc en contact avec les bras supérieurs 1023B et glisse le long de ces bras. Du fait de la forme évasée de la jupe, les bras supérieurs 1023B sont progressivement rapprochés l'un de l'autre, jusqu'à atteindre la configuration d'obturation de l'obturateur amont 1B. Cette première phase du déplacement du second élément 11 n'a pas d'effet sur la configuration de l'obturateur aval 1A, qui reste en configuration d'obturation.

A partir d'une certaine position du second élément par rapport au premier élément, s'engage une seconde phase du déplacement du second élément 11, dans laquelle la jupe 112 exerce une sollicitation sur les bras 1023B, 1023A qui engendre un pivotement élastique des bras 1023A tendant à les éloigner l'un de l'autre, jusqu'à atteindre la configuration d'ouverture de l'obturateur aval 1A. Pendant cette seconde phase, l'obturateur amont 1B reste dans sa configuration d'obturation. A l'issue de cette seconde phase, l'objet contenu dans la chambre est libéré hors du dispositif et ainsi mis à disposition de l'utilisateur.

Lorsque l'utilisateur relâche la pression sur le récipient, un moyen de rappel (non illustré) ramène le récipient 3 vers le haut et donc le second élément 11 vers la position de repos illustrée sur la figure 3C. Le déplacement du second élément vers le haut comprend également deux phases : une première phase au cours de laquelle l'obturateur aval 1A revient en position d'obturation (l'obturateur amont 1B restant en configuration d'obturation) puis une seconde phase au cours de laquelle l'obturateur 1B revient en position d'ouverture (l'obturateur aval 1A restant en configuration d'obturation). Un nouvel objet peut donc être introduit dans la chambre 101 en vue d'une nouvelle opération de distribution qui peut être réalisée immédiatement (par exemple si la dose à délivrer à l'utilisateur comprend plusieurs objets qui ne peuvent être libérés simultanément) ou ultérieurement.

Dans tous les cas, la configuration des premier et second éléments est telle qu'une situation dans laquelle à la fois l'obturateur amont et l'obturateur aval seraient dans une configuration d'ouverture de la chambre ne peut se produire.

Pour sécuriser le dispositif, celui-ci comprend un verrou mobile entre un état de verrouillage dans lequel ledit verrou coopère avec l'obturateur aval pour obturer partiellement le conduit de distribution, et un état de déverrouillage dans laquelle le verrou est escamoté hors du conduit de distribution.

Le verrou est agencé à l'intérieur du dispositif de distribution, de manière non accessible à un utilisateur. Ainsi, le verrou ne peut pas être actionné par un utilisateur depuis l'extérieur du dispositif de distribution. Comme on le verra plus bas, le verrou est adapté pour coopérer avec un actionneur agencé dans un boîtier « intelligent » qui ne commande l'actionneur que dans des situations où l'utilisateur est autorisé à prélever des objets.

Selon un mode de réalisation, le verrou 4 comprend deux palettes 40 opposées comprenant chacune un doigt 41 transversal, ledit doigt s'étendant par exemple perpendiculairement à la palette. Lesdites palettes 40 sont mobiles entre une position de verrouillage dans laquelle leurs doigts 41 sont engagés dans le conduit de distribution de manière à l'obturer partiellement, et une position escamotée dans laquelle lesdits doigts 41 sont situés hors du conduit de distribution, libérant le passage des objets. De préférence, dans la position de verrouillage, les doigts 41 sont non jointifs. Dans cette position de verrouillage, le passage des objets est impossible même si les obturateurs sont ouverts.

Ledit verrou est configuré pour que la position d'un objet présent dans la chambre 101 ne soit pas affectée par un changement d'état du verrou. En pratique, les doigts des palettes sont agencées dans le même plan que l'obturateur aval 1A, ou dans un plan parallèle suffisamment proche de l'obturateur aval 1A pour qu'un objet ne puisse pas être emprisonné entre le verrou et l'obturateur, ni endommagé par la fermeture de l'obturateur. Par exemple, selon la forme des objets, une distance de moins de 3 mm entre le verrou est l'obturateur est privilégiée.

Par ailleurs, le verrou 4 n'interfère pas avec l'obturateur aval 1A, de sorte que le verrou 4 et l'obturateur aval 1A peuvent être actionnés indépendamment l'un de l'autre.

Chaque palette 40 est mobile en rotation autour d'un axe parallèle à l'axe X. Chaque palette est actionnable par un actionneur respectif agencé dans le boîtier susmentionné.

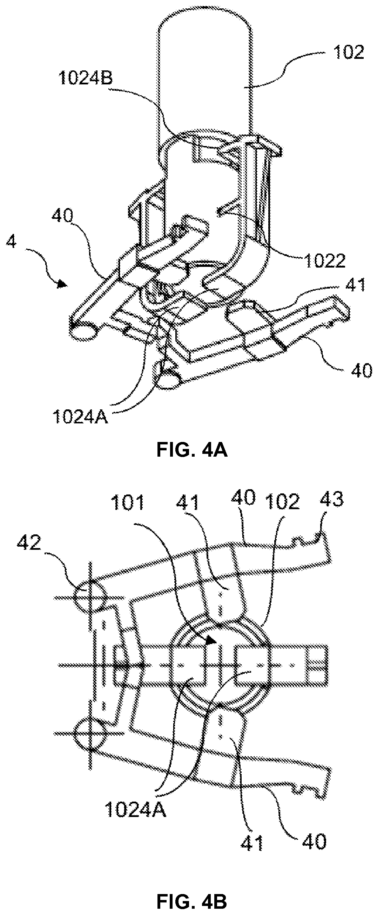

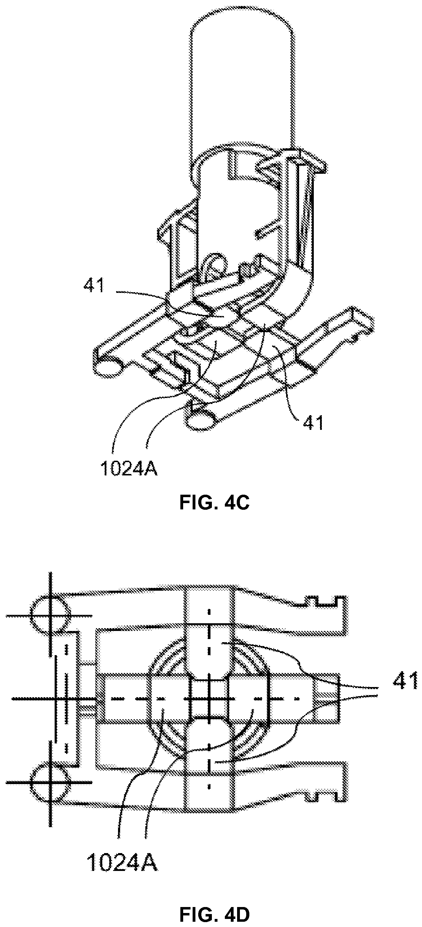

Les figures 4A à 4D illustrent un premier mode de réalisation du verrou, à l'état escamoté (figures 4A-4B) et à l'état de verrouillage (figures 4C-4D).

Chaque palette 40 comprend un membre principal droit, s'étendant dans un plan perpendiculaire à l'axe X et présentant à l'une de ses extrémités un axe de pivotement 42 parallèle à l'axe X et à l'extrémité opposée 43 un moyen de liaison à un actionneur (non représenté). La palette comprend également un doigt 41 qui s'étend à partir du membre principal, sensiblement perpendiculairement à celui-ci, dans un plan perpendiculaire à l'axe X. Les palettes sont agencées de telle sorte que, dans la position de verrouillage, les doigts 41 soient diamétralement opposés par rapport au conduit 100 et perpendiculaires aux extensions 1024A de l'obturateur aval 1A.

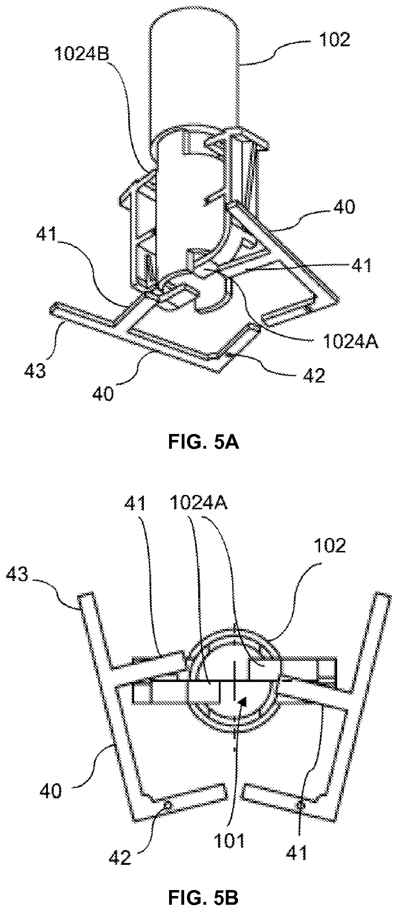

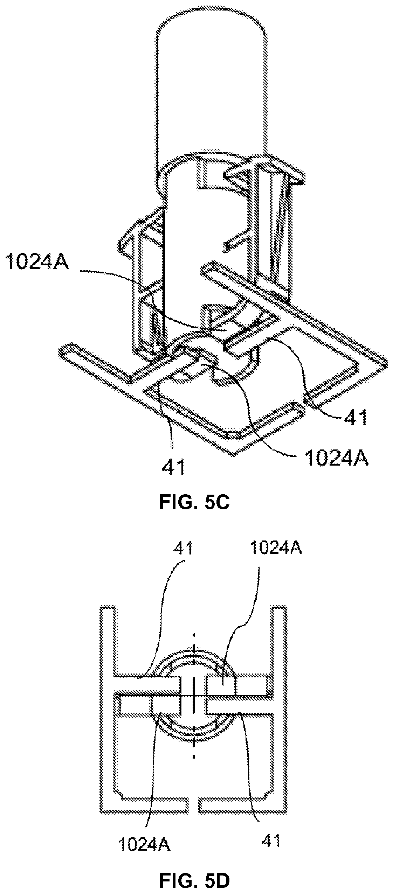

Les figures 5A à 5D illustrent un second mode de réalisation du verrou, à l'état escamoté (figures 5A-5B) et à l'état de verrouillage (figures 5C-5D).

Chaque palette 40 comprend un membre principal droit, s'étendant dans un plan perpendiculaire à l'axe X et présentant à l'une de ses extrémités un axe de pivotement 42 parallèle à l'axe X et à l'extrémité opposée 43 un moyen de liaison à un actionneur (non représenté). La palette comprend également un doigt 41 qui s'étend à partir du membre principal, sensiblement perpendiculairement à celui-ci, dans un plan perpendiculaire à l'axe X. Les palettes sont agencées de telle sorte que, dans la position de verrouillage, les doigts 41 soient parallèles aux extensions 1024A de l'obturateur aval 1A, chaque doigt étant en regard d'une extension opposée.

Le dispositif de comptage et de distribution dont différents modes de réalisation ont été décrits ci-dessus peut se présenter sous la forme d'un composant à assembler sur un pot contenant des objets à distribuer. Le dispositif de distribution présente alors des dimensions permettant de l'adapter sur un contenant existant, tel qu'un pot, un tube, etc. La solidarisation du dispositif de distribution sur le récipient est réalisée par tout moyen approprié, incluant une soudure, un collage, un encliquetage, etc.

De manière alternative, le dispositif de comptage et de distribution peut comprendre le pot, par exemple en faisant en sorte que l'un des éléments soit fabriqué d'un seul tenant avec le pot.

### Boîtier

Le contrôle de la distribution des objets est assuré par un boîtier qui comprend une unité de contrôle et au moins un actionneur piloté par ladite unité de contrôle, le verrou ne pouvant être actionné par l'actionneur qu'après que le dispositif de comptage et de distribution a été inséré dans le boîtier et que l'actionneur a reçu une instruction de déverrouillage de l'unité de contrôle. Une telle instruction intervient dès lors que l'unité de contrôle a déterminé qu'un ou plusieurs objets devaient être distribués. Pour effectuer cette détermination, l'unité de contrôle se base sur des données relatives au traitement, telles que l'identité du patient, le programme planifié de prise du traitement (date, heure, quantité d'objets par prise, etc.).

Le dispositif de distribution et le boîtier forment ensemble un distributeur sécurisé d'une dose d'un traitement thérapeutique.

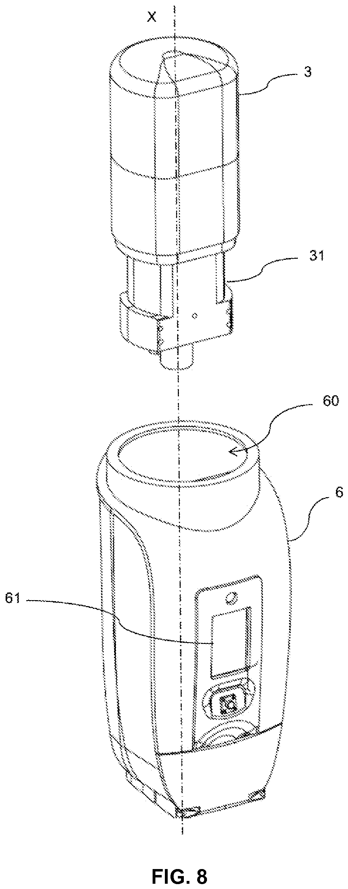

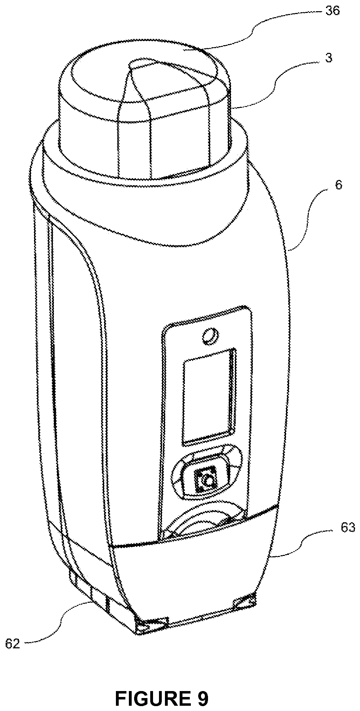

Les figures 8 et 9 sont des vues en perspective du boîtier 6 et d'un récipient 3 comprenant le dispositif de distribution (tel que déjà décrit en référence à la figure 3C), respectivement avant et après assemblage.

De préférence, le dispositif de distribution et le boîtier sont suffisamment compacts et légers pour que le distributeur soit portatif, c'est-à-dire transportable d'une seule main par un utilisateur.

L'unité de contrôle comprend typiquement un processeur ainsi qu'une mémoire dans laquelle sont enregistrées les données relatives au traitement et/ou un moyen d'interrogation d'un serveur distant disposant de ces données. De manière avantageuse, l'unité de contrôle comprend à la fois une mémoire dans laquelle sont enregistrées ces données (par exemple par un praticien) et un moyen d'interrogation d'un serveur sur lequel lesdites données sont également accessibles, ce qui permet si nécessaire d'effectuer une double vérification avant d'autoriser la distribution d'un ou plusieurs objets.

Selon un mode de réalisation, l'actionneur est un actionneur linéaire.

Selon un autre mode de réalisation, l'actionneur est un actionneur rotatif couplé à une came.

De manière particulièrement avantageuse, le boîtier comprend un ou plusieurs capteurs couplés à l'unité de contrôle, choisis en particulier parmi les différents capteurs décrits ci-après.

Un premier capteur est un capteur adapté pour détecter la présence ou le passage d'un objet dans la chambre et/ou dans une portion du conduit située en aval du second obturateur.

Un second capteur est un capteur adapté pour détecter la configuration d'ouverture ou d'obturation de chaque obturateur.

Un troisième capteur est un capteur adapté pour détecter l'état du verrou.

Lesdits capteurs sont avantageusement des fourches optiques, comprenant un émetteur et un récepteur agencés en regard l'un de l'autre, à une certaine distance dans laquelle est agencé l'élément dont on souhaite déterminer la position ou l'état. De manière avantageuse, le boîtier comprend des guides de lumière agencés d'une part entre l'émetteur et un côté de l'élément, et d'autre part entre le récepteur et le côté opposé de l'élément. Ces guides de lumière permettent de positionner l'émetteur/récepteur à un seul emplacement du boîtier pour tous les capteurs, ce qui augmente la compacité du système. Naturellement, l'homme du métier pourra choisir une autre technologie connue de capteur adaptée à cet usage.

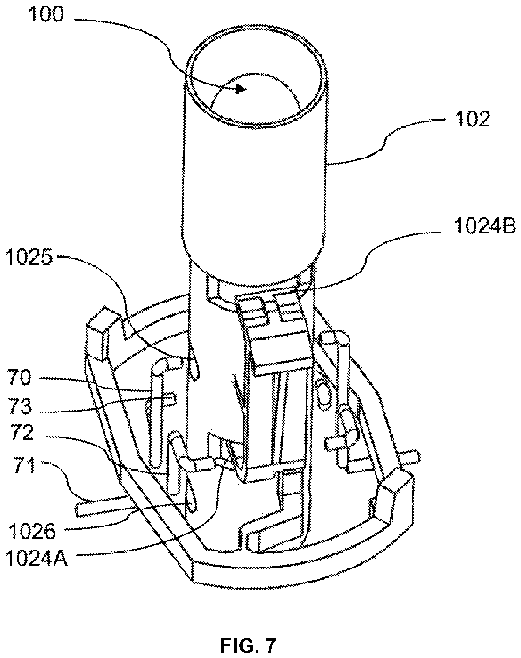

La figure 7 illustre un mode de réalisation incluant de tels guides de lumière. Le fût 102 peut comprendre deux orifices : un orifice 1025 débouchant dans la chambre 101, et un orifice 1026 débouchant dans une portion du conduit de distribution 100 en aval de l'obturateur 1A. Un guide de lumière 70 est agencé entre un capteur optique (non représenté) et l'orifice 1025, afin de permettre de détecter la présence d'un objet dans la chambre 101. Un guide de lumière 71 est agencé entre un capteur optique (non représenté) et l'orifice 1026, afin de permettre de détecter le passage d'un objet au travers de l'obturateur aval. Un guide de lumière 72 est agencé en regard de chaque extension 1024A, afin de permettre de déterminer la configuration (ouverture ou obturation) de l'obturateur 1A. Enfin, un guide de lumière 73 est agencé en regard du doigt de chaque palette, afin de permettre de déterminer l'état (escamoté ou verrouillé) du verrou.

De manière avantageuse, le boîtier comprend en outre un dispositif d'identification du patient couplé à l'unité de contrôle, l'unité de contrôle étant configurée pour n'activer l'actionneur que si le patient est identifié par le dispositif d'identification. Ce dispositif d'identification peut comprendre un dispositif biométrique, et/ou une interface de saisie d'un code d'identification. De tels dispositifs sont connus et ne seront donc pas décrits en détail dans le présent texte.

De manière particulièrement avantageuse, le distributeur comprend un accéléromètre ou un gyroscope couplé à l'unité de contrôle. L'unité de contrôle est configurée pour déterminer l'orientation du distributeur à partir des données de mesure dudit accéléromètre ou gyroscope et pour activer l'actionneur uniquement si le conduit de distribution est orienté sensiblement verticalement vers le bas pour permettre la distribution d'un objet. En d'autres termes, les deux obturateurs doivent être alignés verticalement et le second obturateur doit être situé sous le premier obturateur. Ceci permet d'empêcher l'introduction d'un objet dans le dispositif de distribution alors que le second obturateur est ouvert.

Selon un mode de réalisation, le distributeur comprend un capteur de température et une mémoire (qui peut être la mémoire de l'unité de contrôle susmentionnée) configurée pour enregistrer les données de mesure dudit capteur. Cet enregistrement de la température au cours du temps peut permettre de vérifier que le médicament n'a pas été exposé à une température susceptible de le dégrader. L'unité de contrôle peut être configurée pour émettre une alarme si la température dépasse un plafond prédéfini.

Le distributeur peut en outre comprendre une horloge, l'information concernant la date et l'heure étant transmise au processeur pour le suivi du programme planifié de prise du traitement.

Selon un mode de réalisation, le boîtier comprend une source d'énergie (pile ou batterie, avantageusement rechargeable) alimentant l'unité de contrôle. Ainsi, le distributeur est autonome et peut être déplacé aisément par l'utilisateur.

Selon un mode d'utilisation particulièrement préféré, le boîtier comprend une base 62 sensiblement plane adaptée pour être posée de manière stable sur un support lors de la distribution des objets. Ainsi, une fois que le dispositif de distribution a été mis en place dans le boîtier, l'utilisateur a simplement à exercer une pression verticale vers le bas au moyen d'une seule main (l'autre main restant libre ou servant seulement à maintenir le boîtier) sur l'élément mobile du dispositif de distribution (en l'espèce, le sommet 36 du récipient 3) pour le faire coulisser par rapport à l'élément fixe et pour libérer le nombre d'objets nécessaire. Compte tenu de l'orientation du dispositif de distribution, les objets tombent par gravité dans une partie inférieure du boîtier 6, accessible par une trappe 63.

Bien que les différents capteurs, l'accéléromètre ou le gyroscope décrits plus haut puissent être intégrés au dispositif de distribution, ils seront de préférence intégrés au boîtier.

Ainsi, le dispositif de distribution est avantageusement jetable, c'est-à-dire qu'une fois vidé de l'ensemble des objets, il ne peut être démonté puis rempli de nouveaux objets. Le distributeur étant formé de pièces moulées en matière plastique, il est particulièrement peu onéreux et aisé à recycler.

En revanche, le boîtier 6, qui comprend le processeur et les autres composants électroniques de l'unité de contrôle, l'actionneur, les différents capteurs, le dispositif d'identification, l'accéléromètre et/ou le gyroscope, qui peuvent être relativement onéreux et potentiellement complexes à recycler, est réutilisable.

### Fonctionnement du distributeur

Le distributeur fonctionne de la manière suivante.

L'utilisateur se munit d'un récipient 3 comprenant le dispositif de distribution et les objets constituant le traitement, qui sont conditionnés en vrac dans le récipient. L'obturateur aval est en configuration d'obturation et le verrou est en position de verrouillage, de sorte qu'ils obturent ensemble au moins partiellement le conduit de distribution.

L'utilisateur insère le récipient 3 dans le boîtier 6.

A l'issue de cette opération, chaque palette du verrou se trouve en regard de l'actionneur respectif présent dans le boîtier.

En fonction des données sur le traitement qui ont préalablement été enregistrées dans la mémoire de l'unité de contrôle, l'unité de contrôle peut déclencher ou non une commande d'ouverture du verrou dans sa position escamotée. Une telle commande peut par exemple être déclenchée si l'utilisateur s'est dûment identifié au moyen d'un dispositif d'identification tel que susmentionné, et si l'heure indiquée par l'horloge est dans une plage de temps prévue dans le plan de prise du traitement. Dans le cas contraire, par exemple si l'utilisateur n'a pas été reconnu par le dispositif d'identification, ou si l'heure indiquée par l'horloge est en-dehors de la ou les plage(s) de temps prévue(s) dans le plan de prise du traitement, l'unité de contrôle ne déclenche aucune commande de l'actionneur.

L'utilisateur presse le sommet du récipient 3, qui est rigidement lié au second élément du dispositif de comptage et de distribution vers le bas, puis le relâche, ce qui déclenche la séquence de fonctionnement des obturateurs et permet, si le verrou est dans sa position escamotée, la sortie d'un ou plusieurs objets.

Les capteurs permettent avantageusement de compter le nombre d'objets sortant de la chambre.

Une fois que le nombre d'objets à délivrer a été atteint, l'unité de contrôle envoie à l'actionneur une commande pour ramener le verrou dans sa position de verrouillage.

Par conséquent, même si l'utilisateur actionne à nouveau le dispositif, aucun objet supplémentaire n'en sortira.

### REFERENCES

WO 2015/121353

## Revendications

1. Dispositif (1) de comptage et de distribution d'objets (2), comprenant deux éléments (10, 11) mobiles en coulissement l'un par rapport à l'autre,
- un premier élément (10) comprenant un conduit (100) de distribution des objets à compter et distribuer, ledit conduit (100) s'étendant dans la direction de coulissement,
- le second élément (11) coopérant avec le premier pour former deux obturateurs (1A, 1B) délimitant dans le conduit de distribution (100) une chambre (101) adaptée pour contenir un nombre déterminé desdits objets,
lesdits obturateurs (1A, 1B) étant aptes à adopter, selon la position relative desdits premier et second éléments :
• une configuration dite d'ouverture de la chambre, dans laquelle chaque obturateur définit un orifice de dimension adaptée au passage d'un objet à compter et distribuer, et
• une configuration dite d'obturation de la chambre, dans laquelle ledit orifice présente une dimension insuffisante pour le passage d'un objet, chaque obturateur présentant deux portions biseautées en vis-à-vis non jointives dans ladite configuration d'obturation,
les premier et second éléments (10, 11) étant agencés pour procurer, par coulissement relatif, une séquence de fonctionnement des obturateurs dans laquelle :
(i) un premier obturateur est en configuration d'ouverture tandis que le second obturateur est en configuration d'obturation de la chambre (101),
(ii) le premier et le second obturateurs sont tous deux en configuration d'obturation de la chambre (101),
(iii) le premier obturateur est en configuration d'obturation tandis que le second obturateur est en configuration d'ouverture de la chambre (101),
(iv) le premier et le second obturateurs sont tous deux en configuration d'obturation de la chambre (101),
ledit dispositif étant **caractérisé en ce qu'**il est adapté pour être inséré dans un boîtier (6) comprenant une unité de contrôle et au moins un actionneur (5) piloté par ladite unité de contrôle, et **en ce qu'**il comprend un verrou (4) déplaçable par l'actionneur entre un état de verrouillage dans lequel ledit verrou obture partiellement le conduit de distribution (100), et un état de déverrouillage dans laquelle le verrou est escamoté hors du conduit de distribution (100), ledit verrou étant configuré pour que la position d'un objet présent dans la chambre (101) ne soit pas affectée par un changement d'état du verrou.

2. Dispositif selon la revendication 1, dans lequel le verrou est agencé dans un même plan que les portions biseautées du second obturateur (1A).

3. Dispositif selon l'une des revendications 1 ou 2, dans lequel le verrou comprend deux palettes (40) pivotantes adaptées pour être couplées à un actionneur respectif.

4. Dispositif selon l'une des revendications 1 à 3, dans lequel le premier élément (10), le second élément (11) et le verrou (4) sont chacun réalisés d'un seul tenant, le dispositif (10) étant uniquement constitué desdits premier et second éléments et du verrou.

5. Récipient comprenant le dispositif de distribution selon l'une des revendications 1 à 4 et un pot contenant les objets couplé de manière inamovible audit dispositif de distribution.

6. Récipient selon la revendication 5, dans lequel les objets sont des granules homéopathiques, des gélules, des comprimés, des capsules conditionnés en vrac dans le pot.

7. Distributeur sécurisé d'une dose d'un traitement thérapeutique se présentant sous la forme d'un ou plusieurs objets, ledit distributeur comprenant un boîtier (6) et un récipient (3) selon l'une des revendications 5 ou 6 coopérant avec ledit boîtier, dans lequel le boîtier (6) comprend :
- une unité de contrôle comprenant un processeur et une mémoire dans laquelle sont enregistrées des données relatives au traitement,
- un actionneur (5) piloté par ladite unité de contrôle pour déplacer le verrou (4) dans sa position de verrouillage ou dans sa position escamotée.

8. Distributeur selon la revendication 7, dans lequel l'actionneur est un actionneur linéaire ou un actionneur rotatif.

9. Distributeur selon l'une des revendications 7 à 8, dans lequel le boîtier (6) comprend au moins un capteur couplé à l'unité de contrôle et adapté pour détecter le passage d'un objet dans la chambre et/ou dans une portion du conduit située en aval du second obturateur.

10. Distributeur selon l'une des revendications 7 à 9, dans lequel le boîtier (6) comprend au moins un capteur couplé à l'unité de contrôle et adapté pour détecter la configuration d'ouverture ou d'obturation de chaque obturateur.

11. Distributeur selon l'une des revendications 7 à 10, dans lequel le boîtier (6) comprend en outre un dispositif d'identification du patient couplé à l'unité de contrôle, notamment un dispositif d'identification comprenant un dispositif biométrique ou une interface de saisie d'un code d'identification l'unité de contrôle étant configurée pour activer l'actionneur seulement si le patient est identifié par le dispositif d'identification.

12. Distributeur selon l'une des revendications 7 à 11, comprenant un accéléromètre ou un gyroscope couplé à l'unité de contrôle, dans lequel l'unité de contrôle est configurée pour déterminer l'orientation du distributeur à partir des données de mesure dudit accéléromètre ou gyroscope et pour activer l'actionneur uniquement si le conduit de distribution est orienté sensiblement verticalement et que la sortie du conduit de distribution est dirigée vers le bas.

13. Distributeur selon l'une des revendications 7 à 12, comprenant un capteur de température et une mémoire configurée pour enregistrer les données de mesure dudit capteur.

14. Distributeur selon l'une des revendications 7 à 13, dans lequel le boîtier (6) comprend une source d'énergie alimentant l'unité de contrôle.

15. Distributeur selon l'une des revendications 7 à 14, dans lequel le boîtier (6) comprend une base (62) sensiblement plane adaptée pour être posée sur un support lors de la distribution des objets.

## Patentansprüche

1. Vorrichtung (1) zum Zählen und Ausgeben von Gegenständen (2), mit zwei Elementen (10, 11), die relativ zueinander verschiebbar sind,
- ein erstes Element (10), das einen Kanal (100) zur Verteilung der zu zählenden und zu verteilenden Gegenstände umfasst, wobei sich der Kanal (100) in Schieberichtung erstreckt,
- das zweite Element (11), das mit dem ersten zusammenarbeitet, um zwei Verschlüsse (1A, 1B) zu bilden, die in der Verteilungsleitung (100) eine Kammer (101) begrenzen, die geeignet ist, eine bestimmte Anzahl der genannten Gegenstände aufzunehmen,
wobei die Vewrschlüsse (1A, 1B) in Abhängigkeit von der relativen Position des ersten und zweiten Elements in der Lage sind:
• eine sogenannte Kammeröffnungskonfiguration, bei der jeder Verschluss eine Öffnung in geeigneter Größe definiert, um ein zu zählendes und zu dosierendes Gegenstand durchzuführen, und
• eine sogenannte Verschlusskonfiguration der Kammer, wobei die Öffnung eine unzureichende Größe für das Durchdringen eines Gegenstands aufweist, wobei jeder Verschluss zwei gegenüberliegende, nicht angrenzende abgeschrägte Abschnitte in der Verschlusskonfiguration aufweist,
wobei das erste und das zweite Element (10, 11) so angeordnet sind, dass sie durch relatives Verschieben eine Funktionssequenz der Obturatoren bewirken, wobei:
(i) ein erster Verschluss in der Öffnungskonfiguration ist, während der zweite Verschluss in der Verschlusskonfiguration der Kammer (101) ist,
(ii) sowohl der erste als auch der zweite Verschluss eine Verschlusskonfiguration der Kammer (101) haben,
(iii) der erste Verschluss in der Verschlusskonfiguration ist, während der zweite Verschluss in der Öffnungskonfiguration der Kammer (101) ist,
(iv) sowohl der erste als auch der zweite Verschluss eine Verschlusskonfiguration der Kammer (101) haben,
wobei die Vorrichtung **dadurch gekennzeichnet ist, dass** sie zum Einsetzen in ein Gehäuse (6) geeignet ist, das eine Steuereinheit und wenigstens ein von der Steuereinheit gesteuertes Stellglied (5) umfasst, und dass sie einen Riegel (4) umfasst, der vom Stellglied zwischen einem Verriegelungszustand, in dem der Riegel den Verteilerkanal (100) teilweise verschließt, und einem Entriegelungszustand, in dem der Riegel aus dem Verteilerkanal (100) herausgeschoben ist, verschiebbar ist, wobei der Riegel so konfiguriert ist, dass die Position eines in der Kammer (101) vorhandenen Gegenstands nicht durch eine Zustandsänderung des Riegels beeinflusst wird.

2. Vorrichtung nach Anspruch 1, wobei der Riegel in einer Ebene mit den abgeschrägten Abschnitten des zweiten Verschlusses (1A) angeordnet ist.

3. Vorrichtung nach einem der Ansprüche 1 oder 2, wobei der Riegel zwei schwenkbare Riegel (40) umfasst, die geeignet sind, mit einem jeweiligen Stellglied gekoppelt zu werden.

4. Vorrichtung nach einem der Ansprüche 1 bis 3, wobei das erste Element (10), das zweite Element (11) und der Riegel (4) jeweils aus einem Stück gefertigt sind, wobei die Vorrichtung (10) nur aus dem ersten und zweiten Element und dem Riegel besteht.

5. Behälter, der die Abgabevorrichtung nach einem der Ansprüche 1 bis 4 und einen Behälter mit den Gegenständen umfasst, der an diese Abgabevorrichtung fest gekoppelt ist.

6. Behälter nach Anspruch 5, wobei die Gegenstände homöopathische Granulate, Kapseln, Tabletten, Kapseln sind, die lose im Topf verpackt sind.

7. Sicherer Dispenser für eine Dosis einer therapeutischen Behandlung in Form eines oder mehrerer Gegenstände, wobei der Dispenser ein Gehäuse (6) und einen Behälter (3) nach einem der Ansprüche 5 oder 6 umfasst, der mit dem Gehäuse zusammenarbeitet, wobei das Gehäuse (6) umfasst:
- eine Steuereinheit mit einem Prozessor und einem Speicher, in dem die Verarbeitungsdaten gespeichert sind,
- ein Stellglied (5), das von der genannten Steuereinheit gesteuert wird, um den Riegel (4) in seine Verriegelungsposition oder in seine eingeklappte Position zu bewegen.

8. Verteiler nach Anspruch 7, wobei der Stellglied ein Linearstellglied oder ein Drehstellglied ist.

9. Verteiler nach einem der Ansprüche 7 bis 8, wobei das Gehäuse (6) wenigstens einen Sensor umfasst, der mit der Steuereinheit gekoppelt und geeignet ist, um das Passieren eines Gegenstands in der Kammer und/oder in einem dem zweiten Verschluss nachgelagerten Abschnitt des Kanals zu erkennen.

10. Verteiler nach einem der Ansprüche 7 bis 9, wobei das Gehäuse (6) wenigstens einen Sensor umfasst, der mit der Steuereinheit gekoppelt und geeignet ist, um die Öffnungs- oder Verschlusskonfiguration jedes Verschlusses zu erfassen.

11. Verteiler nach einem der Ansprüche 7 bis 10, wobei das Gehäuse (6) ferner eine mit der Steuereinheit gekoppelte Patientenidentifikationsvorrichtung, insbesondere eine biometrische Vorrichtung oder eine Schnittstelle zur Eingabe eines Identifikationscodes, umfasst, wobei die Steuereinheit so eingerichtet ist, dass sie das Stellglied nur aktiviert, wenn der Patient durch die Identifikationsvorrichtung identifiziert wird.

12. Verteiler nach einem der Ansprüche 7 bis 11, umfassend einen an die Steuereinheit gekoppelten Beschleunigungsmesser oder Gyroskop, wobei die Steuereinheit so eingerichtet ist, dass sie die Ausrichtung des Verteilers aus den Messdaten des Beschleunigungsmessers oder Gyroskops bestimmt und das Stellglied nur dann aktiviert, wenn der Verteilerkanal im Wesentlichen vertikal ausgerichtet ist und der Ausgang des Verteilerkanals nach unten gerichtet ist.

13. Verteiler nach einem der Ansprüche 7 bis 12, umfassend einen Temperatursensor und einen Speicher, der so eingerichtet ist, dass er die Messdaten des Sensors aufzeichnet.

14. Verteiler nach einem der Ansprüche 7 bis 13, wobei das Gehäuse (6) eine Energiequelle umfasst, die die Steuereinheit versorgt.

15. Verteiler nach einem der Ansprüche 7 bis 14, wobei das Gehäuse (6) eine im Wesentlichen ebene Basis (62) umfasst, die geeignet ist, beim Abgeben der Gegenstände auf einem Träger abgelegt zu werden.

## Claims

1. Device (1) for counting and dispensing objects (2), comprising two elements (10, 11) which can be slid relative to one another,
- a first element (10) comprising a channel (100) for dispensing objects to be counted and dispensed, said channel (100) extending in the sliding direction,
- the second element (11) cooperating with the first element to form two shutters (1A, 1B) delimiting a chamber (101) in the distribution channel (100) adapted to contain a determined number of said objects,
said shutters (1A, 1B) being able to adopt, depending on the relative position of said first and second elements:
• a chamber opening configuration, in which each shutter defines an orifice of a size suitable for the passage of an object to be counted and dispensed, and
• a chamber closure configuration, in which said orifice has a dimension insufficient for the passage of an object, each shutter having two opposing bevelled portions which are not contiguous in said closure configuration,
the first and second elements (10, 11) being arranged to provide, by relative sliding, a shutter operating sequence in which:
(i) a first shutter is in the opening configuration, while the second shutter is in the closed configuration of the chamber (101),
(ii) the first and second shutters are both in the chamber (101) closure configuration,
(iii) the first shutter is in the chamber closure configuration, while the second shutter is in the chamber opening configuration (101),
(iv) the first and second shutters are both in the chamber (101) closure configuration,
said device being **characterized in that** it is adapted to be inserted in a housing (6) comprising a control unit and at least one actuator (5) driven by said control unit, and **in that** it comprises a latch (4) displaceable by the actuator between a locking state in which said latch partially obturates the dispensing channel (100), and an unlocking state in which the latch is retracted from the dispensing channel (100), said latch being configured so that the position of an object present in the chamber (101) is not affected by a change of state of the latch.

2. Device as claimed in claim 1, wherein the latch is arranged in the same plane as the bevelled portions of the second shutter (1A).

3. Device according to one of claims 1 or 2, wherein the latch comprises two pivoting vanes (40) adapted to be coupled to a respective actuator.

4. Device according to any of claims 1 to 3, wherein the first element (10), the second element (11) and the latch (4) are each made in one piece, the device (10) consisting solely of said first and second elements and the latch.

5. Container comprising the dispensing device according to one of claims 1 to 4 and a pot containing the objects irremovably coupled to said dispensing device.

6. Container according to claim 5, in which the objects are homeopathic granules, capsules, tablets, capsules packed loose in the pot.

7. Secure dispenser of a dose of a therapeutic treatment in the form of one or more objects, said dispenser comprising a casing (6) and a container (3) according to one of claims 5 or 6 cooperating with said casing, wherein the casing (6) comprises:
- a control unit comprising a processor and a memory in which processing data are stored,
- an actuator (5) driven by said control unit to move the bolt (4) into its locked or retracted position.

8. Dispenser according to claim 7, wherein the actuator is a linear actuator or a rotary actuator.

9. Dispenser according to one of claims 7 to 8, wherein the housing (6) comprises at least one sensor coupled to the control unit and adapted to detect the passage of an object into the chamber and/or into a portion of the channel located downstream of the second shutter.

10. Dispenser according to one of claims 7 to 9, wherein the housing (6) comprises at least one sensor coupled to the control unit and adapted to detect the opening or closing configuration of each shutter.

11. Dispenser according to one of claims 7 to 10, in which the housing (6) further comprises a patient identification device coupled to the control unit, in particular a biometric device or an interface for entering an identification code, the control unit being configured to activate the actuator only if the patient is identified by the identification device.

12. Dispenser according to any of claims 7 to 11, comprising an accelerometer or gyroscope coupled to the control unit, wherein the control unit is configured to determine the orientation of the dispenser from the measurement data of said accelerometer or gyroscope and to activate the actuator only if the dispenser channel is oriented substantially vertically and the outlet of the dispenser channel is directed downwards.

13. Dispenser according to one of claims 7 to 12, comprising a temperature sensor and a memory configured to record measurement data from said sensor.

14. Dispenser according to one of claims 7 to 13, wherein the housing (6) comprises an energy source supplying the control unit.

15. Dispenser according to one of claims 7 to 14, wherein the housing (6) comprises a substantially flat base (62) adapted to be placed on a support when dispensing objects.
